# EUROPÄISCHE PATENTSCHRIFT

(11) **EP 2 257 513 B1**
(45) Veröffentlichungstag und Bekanntmachung des Hinweises auf die Patenterteilung: **17.08.2011**
(21) Anmeldenummer: 09715609.5
(22) Anmeldetag: 27.02.2009
(51) Int. Cl.: C07C 29/56, C07C 33/03

(54) **VERFAHREN ZUR ISOMERISIERUNG VON OLEFINISCH UNGESÄTTIGTEN ALKOHOLEN**
METHOD FOR ISOMERIZING OLEFINICALLY UNSATURATED ALCOHOLS
PROCÉDÉ D'ISOMÉRISATION D'ALCOOLS OLÉFINIQUEMENT INSATURÉS

(30) Priorität: 28.02.2008 DE 102008011767
(43) Veröffentlichungstag der Anmeldung: 08.12.2010
(73) Patentinhaber: BASF SE, 67056 Ludwigshafen (DE)
(72) Erfinder: LIMBACH, Michael, 67551 Worms (DE); TELES, Joaquim Henrique, 67166 Otterstadt (DE); ABDALLAH, Radwan, 67063 Ludwigshafen (DE); MÄURER, Torsten, 67245 Lambsheim (DE); JOHANN, Thorsten, 67117 Limburgerhof (DE); DANZ, Manuel, 69214 Eppelheim (DE)
(86) Internationale Anmeldenummer: PCT/EP2009/052384
(87) Internationale Veröffentlichungsnummer: WO 2009/106622

(56) Entgegenhaltungen:
- WO-A1-2008/098774
- DE-A1- 2 751 766

## Beschreibung

Die vorliegende Erfindung betrifft ein Verfahren zur Isomerisierung von olefinisch ungesättigten Alkoholen in sauerstoffhaltiger Atmosphäre an einem geträgerten Katalysator. Weiterhin betrifft die Erfindung die Verwendung von geträgerten Katalysatoren zur Isomerisierung von olefinisch ungesättigten Alkoholen.

Die Isomerisierung olefinisch ungesättigter Alkohole, z.B. von 3-Methylbut-3-en-1-ol (Isoprenol) zu 3-Methylbut-2-en-1-ol (Prenol) in flüssiger Phase ist bereits beschrieben. In allen Fällen wird die Umsetzung unter einem geringen Strom von Wasserstoff zur Erhöhung der Katalysatorlebenszeit ausgeführt (S. B. Kogan, M. Kalya, N. Froumin, Appl. Cata/. A: General 2006, 27, 231-236). Nach den Angaben dieser Schrift deaktivieren die beschriebenen Edelmetallkatalysatoren in Abwesenheit von Wasserstoff besonders schnell und irreversibel. Selbst unter optimalen Bedingungen nach dieser Schrift ist der mit diesen Katalysatoren erzielte Umsatz relativ gering, wenn hohe Selektivitäten in Richtung Prenol erzielt werden sollen. Bei geringen Umsätzen muss zum wirtschaftlichen Betreiben des Prozesses das nicht umgesetzte Isoprenol abgetrennt und wieder eingesetzt werden. Der Einsatz von Wasserstoff bei der Isomerisierung von Isoprenol ist oft nachteilig, da besonders an auf Silica geträgerten Edelmetallkatalysatoren Nebenreaktionen wie die Hydrierung der Doppelbindung zum gesättigten System beobachtet werden. Dies wird durch die kostspielige Stabilisierung der kationischen Palladium-Zentren durch Dotierung mit Selen oder Cer zu verhindern versucht.

Die US 4,117,016 beschreibt u. a. die Isomerisierung ungesättigter Alkohole in der Flüssigphase in Gegenwart von katalytischen Mengen eines homogenen Ruthenium-Hydrido-Komplexes in Gegenwart eines Liganden und schließt die Gegenwart selbst von Spuren an Sauerstoff ausdrücklich aus, um die Katalysatorstabilität zu gewährleisten. Die Isomerisierung von Isoprenol zu Prenol unter Stickstoff unter den in dieser Schrift genannten Bedingungen gelingt nur in wirtschaftlich uninteressanten und geringen Umsätzen.

Die WO 2008/037693 A1 beschreibt u.a. die Isomerisierung von 3-Methyl-3-buten-1-ol (Isoprenol) an einem heterogenen Edelmetallkatalysator zu 3-Methyl-2-buten-1-ol (Prenol) in Gegenwart von Wasserstoff. Der trimetallische Katalysator besteht aus Pd, Se und Te immobilisiert auf Silica. Hier kommt es durch Überhydrierung in der Wasserstoffatmosphäre zur Bildung von bis zu 2,5% Isoamylalkohol. Dessen Abtrennung ist wirtschaftlich aufwändig.

In der DE-A-1901709 wird ein Verfahren zur Herstellung von Buten-2-olen aus Buten-1-olen beschrieben. Als Katalysatoren dienen elementares Pd oder Pd-Verbindungen in einer Wasserstoffatmosphäre. Bei Verwendung von reinem Pd in Gegenwart von Wasserstoff wird jedoch zum Großteil die Doppelbindung der Ausgangsprodukte hydriert und die entsprechende gesättigte Verbindung gebildet.

Die Hydrierung der Doppelbindung ist unerwünscht, da bei manchen Butenolen nur geringe Siedepunktsdifferenzen zwischen nicht umgesetztem Ausgangsprodukt und Hydrierungsprodukt besteht. So liegt der Siedepunkt von 3-Methyl-but-3-en-1-ol bei 131,5°C (101325 Pa oder 1020 mbar) und der des entsprechenden Hydrierungsproduktes bei 130,9°C (101325 Pa oder1020 mbar).

Aus der DE-A 2751766 und der parallelen US 4,310,709 ist bekannt, dass bei der Isomerisierung von 3-Buten-2-olen zu den entsprechenden 2-Buten-1-olen in Gegenwart eines heterogenen Kontaktes auf Basis von Pd und Se auf Kohlenstoff in Gegenwart von Wasserstoff hohe Anteile an Leichtsiedern wie Isopren und Butenen im Sinne einer *retro*-Prins Reaktion gebildet werden.

Aus der EP-A 841090 ist bekannt, dass die Isomerisierung von 3-Buten-1-olen zu 2-Buten-1-olen in Gegenwart von Wasserstoff nur mit geringer Bildung von Leichtsiedem oder Hydrierprodukten gelingt, wenn der heterogene Pd-Katalysator mit 0.001-0,2 Gew.-% Se, Te oder mit einem Gemisch beider Metalle dotiert ist. So lassen sich Selektivitäten von bis zu 94% bei einem Umsatz von 55% erreichen.

Aus dem Can. J. Chem. 1968, 46, 2225-2232 ist die thermische Isomerisierung von ungesättigten Alkoholen ohne Katalysator bekannt. Bei den erforderlichen hohen Temperaturen wird eine teilweise Verharzung der Ausgangsverbindungen beobachtet.

Nach Lehre der JP-A-8268939 wird Prenol in der Gasphase in Gegenwart von katalytischen Mengen MgO zu Isoprenol isomerisiert. Die Temperaturen sind mit 150-300°C sehr hoch und führen zur Zersetzung der labilen Edukte und Produkte. Aus Sicherheitsgründen wird die Reaktion ausdrücklich unter Schutzgas (Stickstoff) ausgeführt. Es lassen sich Selektivitäten von bis zu 98% bei Umsätzen von 64% erreichen.

Trotz mehrerer bekannter Verfahren zur Isomerisierung ungesättigter Alkohole, insbesondere auch von Isoprenol zu Prenol bestand daher Bedarf, ein entsprechendes Verfahren zur Verfügung zu stellen, welches einerseits verfahrenstechnisch einfach durchführbar ist und andererseits gute Umsätze und Selektivitäten in Richtung der gewünschten Produkte mit sich bringt.

Diese Aufgabe wird mit dem erfindungsgemäßen Verfahren gemäß Anspruch 1 gelöst. Bevorzugte Ausführungsformen ergeben sich aus den Unteransprüchen und der nachfolgenden Beschreibung und den Beispielen.

Nach dem erfindungsgemäßen Verfahren werden olefinisch ungesättigte Alkohole isomerisiert, wobei die Isomerisierung an einem geträgerten edelmetallhaltigen Katalysator in sauerstoffhaltiger Atmosphäre durchgeführt wird.

Als Edelmetalle werden bevorzugt Au, Cu, Ag, Pd, Pt, Rh, Ru, W oder Os eingesetzt, wobei Palladium (Pd) oder Gold (Au) oder Mischungen aus Au oder Pd und den anderen genannten Edelmetallen besonders bevorzugt sind.

Bei der Isomerisierung von Isoprenol zu Prenol haben sich als Edelmetalle Pd oder Au oder Mischungen aus Pd und Au als besonders vorteilhaft herausgestellt.

Als Trägermaterial des im erfindungsgemäßen Verfahren eingesetzten Katalysators wird ein Material auf der Basis von Kohlenstoff verwendet. Nur beispielhaft seien für solche Materialien verschiedene Arten von Kohle oder verschiedene Arten von Graphit genannt, wie sie dem Fachmann bekannt und in der Literatur beschrieben sind.

Grundsätzlich eigenen sich als bevorzugte Trägermaterialien auf Basis von Kohlenstoff alle dem Fachmann für derartige Verwendungen bekannten Kohlenstoffmaterialien. Die Trägermaterialien können vorzugsweise in Form von Formkörpern, Granulaten, Strängen, Pellets, Splitt, Tabletten oder Prills eingesetzt werden. Die Oberfläche der Trägermaterialien nach BET (25°C) liegt üblicherweise im Bereich von 1 bis 10000, vorzugsweise von 10 bis 5000 m²/g, ist aber für das erfindungsgemäße Verfahren in den meisten Fällen nicht kritisch.

In jüngerer Vergangenheit haben sich für manche Umsetzungen auch Kohlenstoff-Nanofasern als Katalysatorträger als vorteilhaft herausgestellt, wie sie dem Fachmann bekannt und in der Literatur beschrieben sind.

Nach dem erfindungsgemäßen Verfahren können insbesondere beta, gamma (β,γ-ungesättigte Alkohole zu α,β-ungesättigten Alkoholen isomerisiert werden. Schematisch lässt sich in diesem Fall die Reaktion wie folgt darstellen:

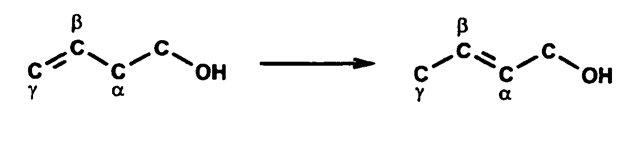

Beispielhaft seien hier als geeignete β,γ-ungesättigte Alkohole solche der allgemeinen Formel I genannt 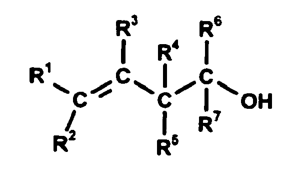 wobei die Substituenten R¹ bis R⁷ unabhängig voneinander jeweils für Wasserstoff, eine C₁-C₈-Alkyl-, eine ggf. substituierte C₃-C₈-Cycloalkyl oder eine ggf. substituierte C₆-C₁₈ Arylgruppe stehen können.

Als Substituenten an den Cycloalkyl- oder Arylgruppen kommen vorzugsweise C₁-C₆-Alkylreste oder C₁-C₆-Alkoxyreste in Frage. Beispielhaft seien für entsprechende Alkohole 3-Buten-1-ol, 3-Penten-1-ol, 3-Methylbut-3-en-1-ol, 3-Hexen-1-ol, 3-Methylpent-3-en-1-ol, 3-Ethylbut-3-en-1-ol und 4-Methylpent-3-en-1-ol genannt.

Eine bevorzugte Gruppe von Alkoholen, die nach dem erfindungsgemäßen Verfahren vorteilhaft isomerisiert werden können, sind die 3-Buten-1-ol-Verbindungen der allgemeinen Formel II

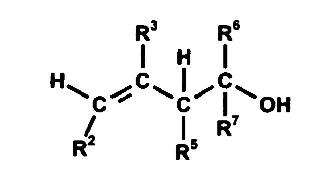

Wobei R², R³, R⁵, R⁶ und R⁷ die vorstehend genannte Bedeutung besitzen.

Vorzugsweise sind die Reste R², R³, R⁵, R⁶ und R⁷ Alkylreste mit 1-6 C-Atomen, die mit einer Hydroxy-, Alkoxy- oder Carboxylgruppe substituiert sein können, besonders bevorzugt Wasserstoff oder Methyl. R⁶ kann auch für einen cycloaliphatischen, araliphatischen oder aromatischen Rest stehen. Als Vertreter dieser Gruppe seien hier 3-Buten-1-ol, 3-Methyl-3-buten-1-ol (Isoprenol), 4-Formyl-3-buten-1-ol, 1,2,3-Trimethyl-3-buten-1-ol, 2-Isobutyl-3-buten-1-ol 3 (2'-hydroxyethyl)-3-buten-1-ol, 1-Hexyl-3-buten-1-ol, 1-Methylen-2-methyl-cyclohexan-3-ol, 1-Methylen-2-ethyl-cyclopentan-3-ol, 1-Methylencyclohexan-3-ol und 1-Methylen-cychoheptan-3-ol genannt.

Die Isomerisierung nach dem erfindungsgemäßen Verfahren wird vorzugsweise in flüssiger Phase durchgeführt. Ein Lösungsmittel ist nicht zwingend erforderlich, es kann auch in den Lösungen des reinen Ausgangsalkohols oder von Mischungen verschiedener Ausgangsalkohole gearbeitet werden.

Wenn ein separates Lösungsmittel eingesetzt wird, haben sich beispielsweise *o-* oder *p-*Xylol oder Ether, wie z.B. Diphenylether, bewährt.

Besonders bevorzugt eignet sich das erfindungsgemäße Verfahren für die Isomerisierung von 3-Methylbut-3-en-1-ol (Isoprenol) zu 3-Methylbut-2-en-1-ol (Prenol). In diesem Fall kann vorzugsweise in reinem Isoprenol oder in einer Mischung aus Isoprenol und einem anderen Alkohol (auch Prenol) oder in einer Mischung aus Isoprenol und einem Aldehyd (z.B. 3-Methyl-but-2-en-1-al oder Prenal) isomerisiert werden.

Das Verfahren kann sowohl in Batch- als auch in kontinuierlicher Fahrweise durchgeführt werden. Bevorzugt wird das Verfahren bei Drücken von weniger als 10 MPa (10⁷ Pa), vorzugsweise weniger als 1 MPa (10⁶ Pa) ausgeführt. Bevorzugte Reaktionstemperaturen liegen bei 0 bis 150, vorzugsweise 10 bis 100 und insbesondere bei der Isomerisierung von Isoprenol nach dem erfindungsgemäßen Verfahren bei 20 bis 80 °C.

Als Hauptnebenprodukt bei der Isomerisierung von Isoprenol nach dem erfindungsgemäßen Verfahren entsteht 3-Methylbut-2-en-1-al (MBA oder Prenal), das durch Oxidation von Prenol unter den oxidativen Bedingungen entsteht. Prenal lässt sich abtrennen und ist eine Vorstufe für Citral, ein kommerziell wichtiges Produkt.

Erfindungsgemäß wird die Umsetzung in einer sauerstoffhaltigen Atmosphäre, durchgeführt. Als entsprechendes Agens lassen sich dabei vorzugsweise reiner Sauerstoff als auch freien Sauerstoff enthaltende Gase, insbesondere solche Gase oder Gasmischungen mit einem Sauerstoffgehalt im Bereich von 2-50 Vol.-%, vorzugsweise von 3-40 Vol.-% und besonders bevorzugt von 7-18 Vol.-% verwenden. Auch Luft kann als leicht zugängliches Oxidationsmedium eingesetzt werden. Alternativ eignet sich auch Wasserstoffperoxid als oxidierendes Agens.

Überraschenderweise werden mit den im erfindungsgemäßen Verfahren eingesetzten edelmetallhaltigen Katalysatoren mit einem Trägermaterial auf Kohlenstoffbasis nur unter oxidativen Bedingungen gute Umsätze und Selektivitäten erzielt, was nach den eindeutigen Lehren des Standes der Technik überraschend und nicht zu erwarten war. Ohne Sauerstoff bzw. unter inerten Bedingungen wie nach dem Stand der Technik verlangt, wird mit diesen geträgerten Katalysatoren keine Isomerisierung in wirtschaftlich interessanten Umfang beobachtet.

Gemäß der Erfindung wird als geträgerter, d.h. auf einem Träger aufgebrachter Katalysator ein Katalysator der ein Edelmetall ausgewählt aus Cu, Ag, Pd, Pt, Rh, Ru, W oder Os enthalten kann, eingesetzt. Die Verwendung von Palladium oder Gold oder Mischungen aus Palladium und Gold oder Mischungen von Palladium oder Gold mit anderen Edelmetallen hat sich dabei in einigen Fällen als vorteilhaft erwiesen. Insbesondere bei der bevorzugten Herstellung von 3-Methylbut-2-en-1-ol lassen sich Reaktionstemperaturen von unter 80°C realisieren, was für die Produktqualität vorteilhaft ist und unerwünschte Nebenreaktionen zurückdrängt.

Das molare Verhältnis von Palladium und/oder Gold zu den anderen Edelmetallen unterliegt dabei keiner besonderen Beschränkung und kann frei gewählt werden.

Bei der Isomerisierung von 3-Methylbut-3-en-1-ol (Isoprenol) hat sich die Verwendung von geträgerten Katalysatoren, die neben Palladium oder Gold ein Edelmetall ausgewählt aus Pt oder Ru enthalten, bewährt.

Der Edelmetallgehalt der geträgerten Katalysatoren, die im erfindungsgemäßen Verfahren eingesetzt werden, unterliegt an sich keiner besonderen Beschränkung und kann im Bereich von 0,1 bis 10 Gew.-%, vorzugsweise im Bereich von 0,4 bis 5 Gew.-% und besonders bevorzugt im Bereich von 0,6 bis 3 Gew.-% liegen.

Als Trägermaterial für den Katalysator eignen sich die in der Literatur beschriebenen und dem Fachmann an sich bekannten Trägermaterialien auf Basis von Kohlenstoff, die vorstehend bereits beschrieben wurden.

Die Herstellung der erfindungsgemäß verwendeten geträgerten Katalysatoren kann nach an sich dem Fachmann bekannten und in der Literatur beschriebenen Verfahren erfolgen. So ist beispielsweise in der EP-A 172 565 oder der EP-A 357 292 ein Verfahren zur Herstellung von geträgerten Silberkatalysatoren beschrieben, das entsprechend angepasst auch für die Herstellung der Katalysatoren der vorliegenden Erfindung verwendet werden kann. Weiter erwähnt sei hier die Herstellung der im erfindungsgemäßen Verfahren eingesetzten Trägerkatalysatoren über das sog. Flammenspritzverfahren (Beschreibung der Technologie z.B. in Army Engineering Manual EM 1110-2-3401) oder aber eine Herstellung in Anlehnung an das in Angew. Chem. Int. Ed. 2007, 47, 138-141 beschriebene Verfahren genannt.

Wegen der einfacheren Herstellungsweise werden Katalysatoren nach dem in der letzten Literaturstelle beschriebenen Verfahren bevorzugt.

Gemäß einer weiteren Ausführungsform betrifft die vorliegende Erfindung die Verwendung von edelmetallhaltigen geträgerten Katalysatoren mit einem Träger auf der Basis von Kohlenstoff zur Isomerisierung von olefinisch ungesättigten Alkoholen, vorzugsweise β,γ-ungesättigten Alkoholen zu α,β-ungesättigten Alkoholen, besonders bevorzugt von Isoprenol zu Prenol, in sauerstoffhaltiger Atmosphäre.

Wegen Einzelheiten hinsichtlich der geeigneten Katalysatoren und der isomerisierbaren Alkohole sei hier auf die vorstehenden entsprechenden Ausführungen im Zusammenhang mit dem erfindungsgemäßen Verfahren verwiesen.

Überraschenderweise lassen sich nach dem erfindungsgemäßen Verfahren olefinisch ungesättigte Alkohole, insbesondere β,γ-ungesättigte Alkohole, unter oxidierenden Bedingungen mit guter Ausbeute und Selektivität isomerisieren. Der Stand der Technik hat für Isomerisierungen dieser Art bislang stets den Ausschluss von Sauerstoff als wesentlich erachtet, d.h. es musste unter inerten oder reduzierenden Bedingungen gearbeitet werden, was verfahrenstechnisch aufwendiger und damit wirtschaftlich nachteilig ist.

Die nachfolgenden Beispiele stellen bevorzugte Ausführungsformen der vorliegenden Erfindung dar und dienen zur weiteren Erläuterung derselben.

### Beispiel 1

In einem Glasautoklaven wurden Isoprenol (103,8 g, 1,21 mol) und der Katalysator (2,40 g, 5,0 Gew.-% Pd/Kohle) zugegeben, es wurden 500 kPa (5 bar) Luft aufgepresst und bei 80°C die Gasphase kontinuierlich ausgetauscht (30 I/h). Es wurde für 0,5 h heftig gerührt. Abtrennen des Katalysators und Analytik des Rückstands ergab folgende Zusammensetzung: 4,32 Gew.-% Prenal, 38,3 Gew.-% Isoprenol, 50,9 Gew.-% Prenol, entsprechend 61,7% Umsatz, 83,4% Selektivität (Prenol), 7,1% Selektivität (Prenal) und 90,5% Selektivität an Wertprodukten.

### Beispiel 2

In einem Glasautoklaven wurden Isoprenol (103,8 g, 1,21 mol) und der Katalysator (2,40 g, 5,0 Gew.-% Pd/Kohle) zugegeben, es wurden 500 kPa (5 bar) Luft aufgepresst und bei 60°C die Gasphase kontinuierlich ausgetauscht (30 I/h). Es wurde für 4,4 h heftig gerührt. Abtrennen des Katalysators und Analytik des Rückstands ergab folgende Zusammensetzung: 8,39 Gew.-% Prenal, 27,1 Gew.-% Isoprenol, 56,2 Gew.-% Prenol, entsprechend 72,9% Umsatz, 78,0% Selektivität (Prenol), 11,6% Selektivität (Prenal) und 89,6% Selektivität an Wertprodukten.

### Beispiel 3

In einem Glasautoklaven wurden Isoprenol (103,8 g, 1,21 mol) in Diphenylether (60 ml) gelöst, so dass sich eine Konzentration von 40 Gew.-% Isoprenol ergab. Der Katalysator (2,40 g, 5,0 Gew.-% Pd/Kohle) wurde zugegeben, es wurde 500 kPa (5 bar) Luft aufgepresst und bei 80°C die Gasphase kontinuierlich ausgetauscht (30 I/h). Es wurde für 4,5 h heftig gerührt. Abtrennen des Katalysators und Analytik des Rückstands ergab folgende Zusammensetzung: 10,4 Gew.-% Prenal, 8,5 Gew.-% Isoprenol, 17,5 Gew.-% Prenol, entsprechend 78,7% Umsatz, 56,4% Selektivität (Prenol), 33,6 % Selektivität (Prenal) und 90,0% Selektivität an Wertprodukten.

### Beispiel 4

In einem Glasautoklaven wurden Isoprenol (103,8 g, 1,21 mol) und der Katalysator (2,40 g, 5,0 Gew.-% Pd/Kohle) zugegeben, es wurde 100 kPa (1 bar) Luft aufgepresst und bei 80°C die Gasphase kontinuierlich ausgetauscht (30 I/h). Es wurde für 4,45 h heftig gerührt. Abtrennen des Katalysators und Analytik des Rückstands ergab folgende Zusammensetzung: 8,22 Gew.-% Prenal, 28,1 Gew.-% Isoprenol, 56,9 Gew.-% Prenol, entsprechend 71,9% Umsatz, 80,0% Selektivität (Prenol), 11,6% Selektivität (Prenal) und 91,6% Selektivität an Wertprodukten.

### Beispiel 5

In einem Glasautoklaven wurden Isoprenol (103,8 g, 1,21 mol) und der Katalysator (2,40 g, 3,4 Gew.-% Pd/Kohle) zugegeben, es wurde Sauerstoff (200 kPa (2 bar)) aufgepresst und bei 80°C für 4,5 h heftig gerührt. Abtrennen des Katalysators und Analytik des Rückstands ergab folgende Zusammensetzung: 5,67 Gew.-% Prenal, 35,7 Gew.-% Isoprenol, 53,8 Gew.-% Prenol, entsprechend 64,3% Umsatz, 84,5% Selektivität (Prenol), 8,9% Selektivität (Prenal) und 93,4% Selektivität an Wertprodukten.

### Beispiel 6 (Vergleichsbeispiel)

In einem Glasautoklaven wurden Isoprenol (103,8 g, 1,21 mol) und der Katalysator (2,40 g, 3,4 Gew.-% Pd/Kohle) zugegeben, es wurde 500 kPa (5 bar) Stickstoff aufgepresst und bei 80°C die Gasphase kontinuierlich ausgetauscht (30 I/h). Es wurde für 5 h heftig gerührt. Abtrennen des Katalysators und Analytik des Rückstands ergab folgende Zusammensetzung: 0,56 Gew.-% Prenal, 96,14 Gew.-% Isoprenol, 0,47 Gew.-% Prenol, entsprechend 3,9% Umsatz, 12,2% Selektivität (Prenol), 14,6% Selektivität (Prenal) und 26,8% Selektivität an Wertprodukten.

### Beispiel 7 (Vergleichsbeispiel)

In einem Glasautoklaven wurden Isoprenol (2,40 g, 27,9 mmol) in *p-*Xylol (120 ml) gelöst, so dass sich eine Konzentration von 2,3 Gew.-% Isoprenol ergibt. Dann wurde der Katalysator (2,40 g, Pd,Se/SiO₂) zugegeben, es wurde 500 kPa Luft (5 bar) aufgepresst und bei 110°C für 1,1 h heftig gerührt. Abtrennen des Katalysators und Analytik des Rückstands ergab folgende Zusammensetzung: 0,01 Gew.-% Prenal, 2,40 Gew.-% Isoprenol, 0,01 Gew.-% Prenol, entsprechend 11,9% Umsatz, 4,8% Selektivität (Prenol), 4,8% Selektivität (Prenal) und 9,6% Selektivität an Wertprodukten.

## Patentansprüche

1. Verfahren zur Isomerisierung von olefinisch ungesättigten Alkoholen an geträgerten edelmetallhaltigen Katalysatoren mit einem Träger auf der Basis von Kohlenstoff, **dadurch gekennzeichnet, dass** die Isomerisierung mit einem geträgerten edelmetallhaltigen Katalysator in sauerstoffhaltiger Atmosphäre durchgeführt wird und
dass β,γ-ungesättigte Alkohole der allgemeinen Formel I zu den entsprechenden α,β-ungesättigten Alkoholen isomerisiert werden 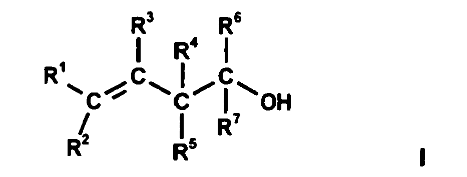 wobei die Substituenten unabhängig voneinander jeweils für Wasserstoff, eine C₁-C₈-Alkyl-, eine ggf. substituierte C₆-C₈-Cycloalkyl- oder eine ggf. substituierte C₆-C₁₈-Arylgruppe stehen können.

2. Verfahren nach Anspruch 1, **dadurch gekennzeichnet, dass** als Edelmetall Au, Cu, Ag, Pd, Pt, Rh, Ru, W, Os oder eine Mischung dieser Metalle eingesetzt wird.

3. Verfahren nach Anspruch 2, **dadurch gekennzeichnet, dass** als Edelmetall Pd oder Au oder eine Mischung aus Pd oder Au und Cu, Ag, Pd, Pt, Ru, Rh, W oder Os eingesetzt wird.

4. Verfahren nach einem der Ansprüche 1 bis 3, **dadurch gekennzeichnet, dass** als sauerstoffhaltige Atmosphäre Sauerstoff, Luft oder ein Sauerstoff enthaltendes Gasgemisch eingesetzt wird.

5. Verfahren nach einem der Ansprüche 1 bis 4, **dadurch gekennzeichnet, dass** 3-Buten-1-ol-Verbindungen der allgemeinen Formel II 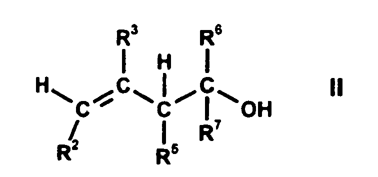 isomerisiert werden.

6. Verfahren nach einem der Ansprüche 1 bis 5, **dadurch gekennzeichnet, dass** 3-Methyl-but-3-en-1-ol (Isoprenol) zu 3-Methyl-but-2-en-1-ol (Prenol) isomerisiert wird.

7. Verwendung eines geträgerten edelmetallhaltigen Katalysators mit einem Träger auf der Basis von Kohlenstoff zur Isomerisierung von olefinisch ungesättigten Alkoholen in sauerstoffhaltiger Atmosphäre, **dadurch gekennzeichnet,**
**dass** β,γ-ungesättigte Alkohole der allgemeinen Formel I zu den entsprechenden α,β-ungesättigten Alkoholen isomerisiert werden 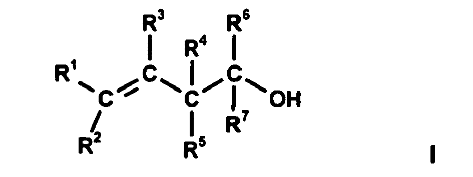 wobei die Substituenten unabhängig voneinander jeweils für Wasserstoff, eine C₁-C₈-Alkyl-, eine ggf. substituierte C₆-C₈-Cycloalkyl- oder eine ggf. substituierte C₆-C₁₈-Arylgruppe stehen können.

8. Verwendung nach Anspruch 7, wobei 3-Methyl-but-3-en-1-ol zu 3-Methyl-but-2-en-1-ol isomerisiert wird.

## Claims

1. A process for isomerizing olefinically unsaturated alcohols over supported noble metal catalysts with a support based on carbon, which comprises performing the isomerization with a supported noble metal catalyst in an oxygenous atmosphere and isomerizing β,γ-unsaturated alcohols of the general formula I to the corresponding α,β-unsaturated alcohols 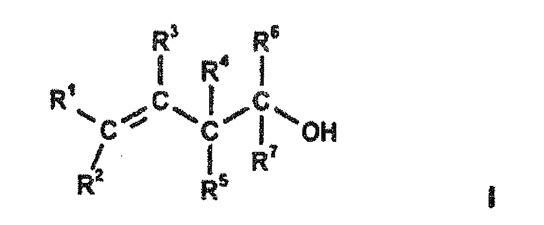 where the substituents may each independently be hydrogen, a C₁-C₈-alkyl group, an optionally substituted C₆-C₈-cycloalkyl group or an optionally substituted C₆-C₁₈-aryl group.

2. The process according to claim 1, wherein the noble metal used is Au, Cu, Ag, Pd, Pt, Rh, Ru, W, Os or a mixture of these metals.

3. The process according to claim 2, wherein the noble metal used is Pd or Au, or a mixture of Pd or Au and Cu, Ag, Pd, Pt, Ru, Rh, W or Os.

4. The process according to any of claims 1 to 3, wherein the oxygenous atmosphere used is oxygen, air or an oxygen-comprising gas mixture.

5. The process according to any of claims 1 to 4, wherein 3-buten-1-ol compounds of the general formula II 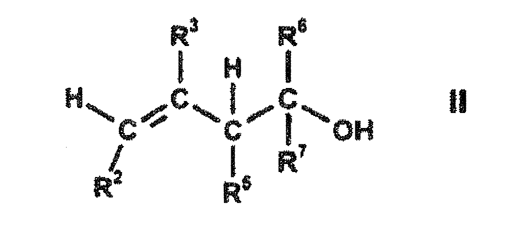 are isomerized.

6. The process according to any of claims 1 to 5, wherein 3-methylbut-3-en-1-ol (isoprenol) is isomerized to 3-methylbut-2-en-1-ol (prenol).

7. The use of a supported noble metal catalyst with a support based on carbon for isomerization of olefinically unsaturated alcohols in an oxygenous atmosphere, which comprises isomerizing β,γ-unsaturated alcohols of the general formula I to the corresponding α,β-unsaturated alcohols 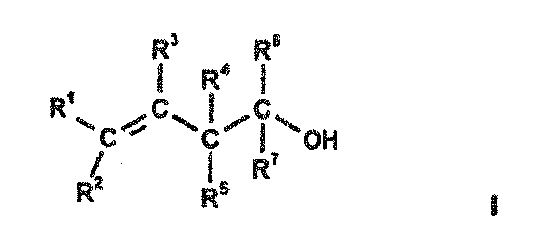 where the substituents may each independently be hydrogen, a C₁-C₈-alkyl group, an optionally substituted C₆-C₈-cycloalkyl group or an optionally substituted C₆-C₁₈-aryl group.

8. The use according to claim 7, wherein 3-methylbut-3-en-1-ol is isomerized to 3-methylbut-2-en-1-ol.

## Revendications

1. Procédé d'isomérisation d'alcools oléfiniquement insaturés sur des catalyseurs supportés contenant un métal noble avec un support à base de carbone, **caractérisé en ce que**
l'isomérisation est réalisée avec un catalyseur supporté contenant un métal noble dans une atmosphère contenant de l'oxygène et
des alcools β,γ-insaturés de formule générale I sont isomérisés en les alcools α,β-insaturés correspondants 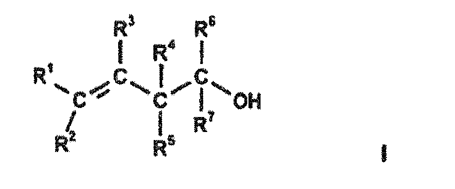 dans laquelle les substituants peuvent représenter indépendamment les uns des autres à chaque fois l'hydrogène, un groupe alkyle en C₁-C₈, un groupe cycloalkyle en C₆-C₈ éventuellement substitué ou un groupe aryle en C₆-C₁₈ éventuellement substitué.

2. Procédé selon la revendication 1, **caractérisé en ce qu'**Au, Cu, Ag, Pd, Pt, Rh, Ru, W, Os ou un mélange de ces métaux est utilisé en tant que métal noble.

3. Procédé selon la revendication 2, **caractérisé en ce que** Pd ou Au ou un mélange de Pd ou Au et de Cu, Ag, Pd, Pt, Ru, Rh, W ou Os est utilisé en tant que métal noble.

4. Procédé selon l'une quelconque des revendications 1 à 3, **caractérisé en ce que** de l'oxygène, de l'air ou un mélange gazeux contenant de l'oxygène est utilisé en tant qu'atmosphère contenant de l'oxygène.

5. Procédé selon l'une quelconque des revendications 1 à 4, **caractérisé en ce que** des composés de 3-butèn-1-ol de formule générale II 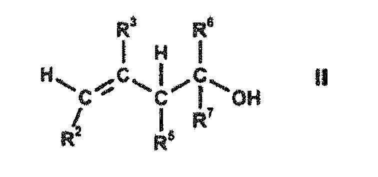 sont isomérisés.

6. Procédé selon l'une quelconque des revendications 1 à 5, **caractérisé en ce que** le 3-méthyl-but-3-èn-1-ol (isoprénol) est isomérisé en 3-méthyl-but-2-èn-1-ol (prénol).

7. Utilisation d'un catalyseur supporté contenant un métal noble avec un support à base de carbone pour l'isomérisation d'alcools oléfiniquement insaturés dans une atmosphère contenant de l'oxygène, **caractérisée en ce que**
des alcools β,γ-insaturés de formule générale I sont isomérisés en les alcools α,β-insaturés correspondants 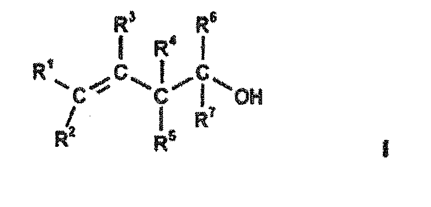 dans laquelle les substituants peuvent représenter indépendamment les uns des autres à chaque fois l'hydrogène, un groupe alkyle en C₁-C₈, un groupe cycloalkyle en C₆-C₈ éventuellement substitué ou un groupe aryle en C₆-C₁₈ éventuellement substitué.

8. Utilisation selon la revendication 7, dans laquelle du 3-méthyl-but-3-èn-1-ol est isomérisé en 3-méthyl-but-2-èn-1-ol.
